# EUROPEAN PATENT APPLICATION

(11) **EP 4 706 710 A1**
(43) Date of publication of application: **11.03.2026**
(21) Application number: 25189200.6
(22) Date of filing: 11.07.2025
(51) Int. Cl.: A61M 15/00

(54) **BUFFERED DRUG AEROSOL DELIVERY DEVICES AND SYSTEMS**

(30) Priority: 10.09.2024 CN 202422216115 U
(71) Applicant: Taian Dalu Medical Instrument Co., Ltd., Taian, Shandong 271000 (CN)
(72) Inventor: LI, Xinze, Taian, 271000 (CN); CUI, Xianwei, Taian, 271000 (CN); WANG, Youjia, Taian, 271000 (CN)
(74) Representative: Gong, Jinping

(57) **Abstract**

The present disclosure discloses a buffered drug aerosol delivery device and system. A chamber and a mouthpiece in the buffered drug aerosol delivery device can be attached to and detached from each other with the aid of first pressing buttons. Further, a chamber and a bottom cover can be attached to and detached from each other with the aid of second pressing buttons. The buffered drug aerosol delivery device is easy to operate, provides a secure connection, and is convenient to clean.

## Description

### TECHNICAL FIELD OF THE DISCLOSURE

The present disclosure relates to a buffered drug aerosol delivery device and system.

### BACKGROUND OF THE DISCLOSURE

A pressurized metered-dose inhaler (MDI) atomizes a metered dose of a drug before ejecting it into the user's mouth and nose. Due to its high speed at the exit of a pressurized MDI, the aerosol tends to deposit at the user' mouth and nose, which is not conducive to its entry into the user's lungs.

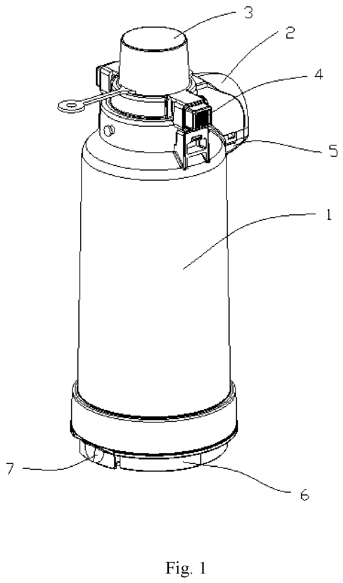

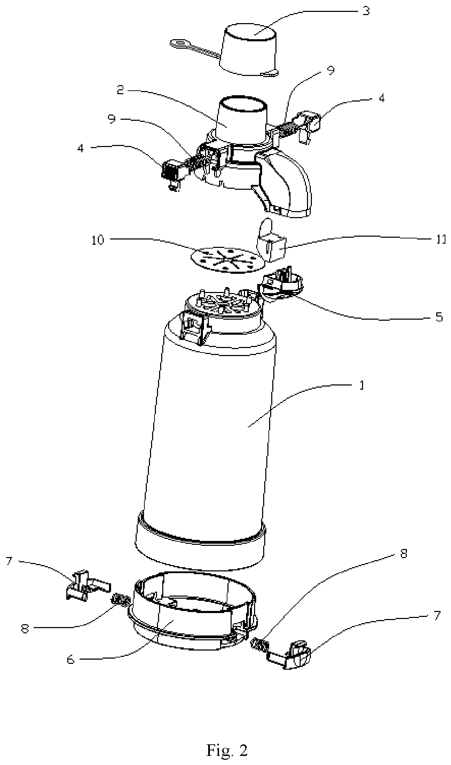

The China utility model patent CN215461014U provides a spacer and inhalation device. An aerosol device delivers a metered dose of aerosol into the spacer, which buffers the aerosol before its entry into the user's mouth and nose, thereby improving the user experience. It can be seen from Figs. 1 and 2 of the patent that its technical solution in essence comprises fitting the bottom of the canister into the top of the base (by a snap-fit joint) and fitting the top of the canister into the bottom of the upper cover (by a snap-fit joint). This enables the detachment of the canister from the base and the attachment of the canister to the base as well as the detachment of the canister from the upper cover and the attachment of the canister to the upper cover. With three detachable components already involved in it, the technical solution further puts forward a preferable way of connection between the upper cover and the mouthpiece that they are fixed to each other by ultrasonic welding.

### SUMMARY OF THE DISCLOSURE

The present disclosure provides a buffered drug aerosol delivery device and system.

One technical solution of the present disclosure is as follows: a buffered drug aerosol delivery device, comprising: a chamber, a mouthpiece, two first pressing buttons, two first springs, a bottom cover, an inhalation controller, and an exhalation controller;
wherein the first pressing button is an integrally formed structure comprising a pressing portion, a first clip, two second clips, and a positioning structure, wherein the first clip is connected to the pressing portion and extends downward, and the two second clips and the positioning structure are all connected to the pressing portion and extend horizontally toward the interior of the buffered drug aerosol delivery device;
the chamber is an integrally formed structure providing a gas passage extending in an up-down direction and comprising two first clip positions arranged on an outer side of the gas passage, wherein the two first clip positions are opposed to each other in a diameter direction of the chamber and are hollow, and a hollow region of the first clip position extends upward and away from the opposed first clip position;
the mouthpiece is an integrally formed structure comprising a main housing and two sleeves, wherein the main housing provides a gas passage extending in an up-down direction, and the two sleeves are arranged on an outer side of the main housing and extend in a diameter direction of the main housing with their respective openings opposed to each other;
the first pressing button, the first clip position, the sleeve, and the first spring are in one-to-one correspondence with each other;
the first spring is located inside the corresponding sleeve, and two ends of the first spring are connected to the main housing of the mouthpiece and the positioning structure of the first pressing button, respectively;
the pressing portion of the first pressing button is located inside the corresponding sleeve, the sleeve is provided, at a bottom thereof, with a void region extending to an end surface thereof that faces away from the other sleeve to allow and restrict the movement of the first pressing button in an extension direction of the corresponding sleeve, and the sleeve is provided, at an end thereof that faces the other sleeve, with two void regions opposed to each other horizontally to secure free ends of the two second clips of the corresponding first pressing button;
when the first pressing button is at a farthest position from the other first pressing button, a free end of the first clip of the first pressing button engages with an upper boundary of the first clip position that faces away from the void region on the end surface of the other first clip position, and when the first pressing button is at a closest position to the other pressing button, the first clip of the first pressing button is movable to be detached from the first clip position (thereby detaching the first pressing button together with the mouthpiece); and
the bottom cover is detachably fixed to a lower end of the chamber, the inhalation controller is configured to allow gas to flow unidirectionally out of the mouthpiece from the chamber through the main housing of the mouthpiece, and the exhalation controller is configured to allow gas to flow unidirectionally out of the mouthpiece from an upper opening of the main housing of the mouthpiece through an internal space of the main housing of the mouthpiece.

Optionally, the chamber comprises a tube-shaped portion and a stepped portion, wherein the tube-shaped portion is tubular and comprises a first opening detachably fixed to the bottom cover, the stepped portion covers a second opening of the tube-shaped portion and comprises a central region protruding upward to form a boss provided, on a top surface thereof, with a first vent communicating with an internal space of the tube-shaped portion, and the first clip position is connected to the stepped portion and configured to be a frame-like structure, is provided, at an upper part thereof, with a third notch, and is provided, on a side thereof that faces away from the boss, with a second notch, wherein the third notch and the second notch communicate with an internal space of the first clip position; and
the main housing of the mouthpiece defines a three-stage stepped through hole, wherein the sleeve of the mouthpiece is opposed to a medium-diameter stage of the three-stage stepped through hole, and the boss of the stepped portion of the chamber is fitted into a section of the main housing that corresponds to the large-diameter stage of the three-stage stepped through hole.

Optionally, the positioning structure of the first pressing button is a cross-shaped positioning rib.

Optionally, the mouthpiece further comprises a first positioning rib connected to the bottom of the sleeve, wherein the first positioning rib extends downward and is inserted into the hollow region of the first clip position.

Optionally, the mouthpiece is provided, at a lower end surface thereof, with a first positioning groove, and the first clip position is connected to a bottom peripheral region of the boss via a fixing rib, wherein a first notch is formed above the fixing rib, and the first positioning groove is mounted onto the fixing rib by passing through the first notch.

Optionally, the buffered drug aerosol delivery device further comprises two second pressing buttons and two second springs;
wherein the second pressing button is an integrally formed structure comprising a pressing plate, a third clip, two fourth clips, and a first positioning post, wherein the first positioning post and the fourth clips are located on the same side of the pressing plate and extend horizontally toward the interior of the buffered drug aerosol delivery device, and the third clip extends upward;
the chamber further comprises two second clip positions located at the first opening of the tube-shaped portion thereof, wherein the second clip position is an arc-shaped flat plate;
the bottom cover comprises an integrally formed rigid portion comprising four first through holes for insertion of the fourth clip of the second pressing button, wherein an edge of the first through hole forms a third clip position configured to prevent the fourth clip from being detached from the bottom cover, and the rigid portion of the bottom cover further comprises two second positioning posts arranged between adjacent ones of the first through holes and extending radially outward;
the second pressing button, the second spring, and the second positioning post are in one-to-one correspondence with each other;
two ends of the second spring cover the corresponding second positioning post and the first positioning post of the corresponding second pressing button respectively and abut against the bottom cover and the pressing plate of the second pressing button;
a free end of the third clip of the second pressing button engages with the second clip position to prevent the bottom cover from being detached axially from the tube-shaped portion; and
the bottom cover comprises, on the outer peripheral surface of a section thereof that faces the tube-shaped portion, two concave sections, wherein the second clip position is located between the two concave sections to prevent the bottom cover from rotating with respect to the tube-shaped portion.

Optionally, the bottom cover comprises a first annular curved plate, a first annular flat plate, a second annular curved plate, and a second annular flat plate, connected in this order from top to bottom, wherein the first annular curved plate defines a first cylindrical internal space, the second annular curved plate defines a second cylindrical internal space, and a central opening region of the second annular flat plate, the second cylindrical internal space, a central opening region of the first annular flat plate, and the first cylindrical internal space communicate, in this order, with each other to form a through hole;
the second annular curved plate comprises two opposed concave sections in one-to-one correspondence with the second pressing buttons, wherein the pressing plate of the second pressing button is opposed to the corresponding concave section of the second annular curved plate, and the first through hole into which the fourth clip of the second pressing button is inserted is arranged at the concave section of the second annular curved plate;
the first annular flat plate comprises two opposed concave sections in one-to-one correspondence with the second pressing buttons, wherein the concave section of the first annular flat plate is configured to avoid the third clip of the second pressing button; and
the first annular curved plate is inserted into the first opening of the tube-shaped portion and comprises two opposed concave sections in one-to-one correspondence with the second pressing buttons, wherein the second clip position is located inside the corresponding concave section of the first annular curved plate to prevent the bottom cover from rotating with respect to the tube-shaped portion.

Optionally, the inhalation controller is a flower-shaped washer arranged on a top surface of the boss of the stepped portion.

Optionally, the exhalation controller is a valve structure, and the main housing of the mouthpiece is provided with a second vent. The valve structure comprises an elastic valve flap configured to cover the second vent. The valve flap is designed as a thin sheet, which is easy to open and close, making the structure responsive.

Another technical solution of the present disclosure is as follows: a buffered drug aerosol delivery system, comprising a pressurized metered-dose inhaler and the buffered drug aerosol delivery device described above.

The present disclosure provides a novel buffered drug aerosol delivery device designed to be capable of being disassembled. The buffered drug aerosol delivery device features simpler disassembly and assembly processes, is more steady when it is in the assembled state, and is easy to clean.

### BRIEF SUMMARY OF THE DRAWINGS

Fig. 1 is a perspective view of the buffered drug aerosol delivery device of the present disclosure in the assembled state.
Fig. 2 is an exploded view of the buffered drug aerosol delivery device of the present disclosure.
Fig. 3 is a perspective view of part of the chamber.
Fig. 4 is a perspective view of the chamber.
Figs. 5 and 6 are perspective views of the mouthpiece.
Figs. 7 and 8 are perspective views of the first pressing button.
Figs. 9 and 10 are perspective views of the second pressing button.
Figs. 11 and 12 are perspective views of the bottom cover.
Fig. 13 is a perspective view of the flower-shaped washer.
Figs. 14 and 15 are perspective views of the mouthpiece clip.
Figs. 16 and 17 are perspective views of the valve structure.
Fig. 18 is a perspective view of the dust cap.
Fig. 19 is a schematic diagram of a partial structure near the mouthpiece of the buffered drug aerosol delivery device.
Fig. 20 is a schematic diagram of a partial structure near the bottom cover of the buffered drug aerosol delivery device.

The reference numerals of the components are as follows:
1-chamber; 12-fixing surface; 13-first insertion surface; 14-first clip position; 15-fixing rib; 16-first fixing post; 17-second clip position; 18-tube-shaped portion; 19-first vent; 1a-first notch; 1b-second notch; 1c-third notch; 1d-plate-shaped region;
2-mouthpiece; 21-oral portion; 22-fixing male snap; 23-first positioning rib; 24-sleeve; 25-vertical rib; 26-second insertion surface; 27-first positioning groove; 28-positioning boss; 29-second positioning rib; h-three-stage stepped through hole; 2a-fourth notch; 2b-fifth notch; 2c-extension portion; 2d-second vent; 2e-second through hole;
3-dust cap; 31-cap body; 32-cap visor; 33-fixing female snap;
4-first pressing button; 41-second clip; 42-first clip; 43-pressing portion; 44-positioning rib;
5-mouthpiece clip; 51-fifth clip; 52-exhaust hole;
6-bottom cover; 61-soft rubber ring; 62-second positioning post ; 63-third clip position; 64-second annular flat plate; 65-second annular curved plate; 66-first annular flat plate; 67-first annular curved plate; 650-first through hole; 670-sixth notch;
7-second pressing button; 71-fourth clip; 72-third clip; 73-first positioning post; 74-pressing plate;
8-second spring;
9-first spring;
10-flower-shaped washer; 101-petal; 102-positioning hole;
11-valve structure; 111-valve flap; 112-stepped surface; 113-second positioning groove; 114-mounting surface.

### DETAIL DESCRIPTION OF THE DISCLOSURE

The present disclosure will be further explained by way of specific embodiments, but the protection scope of the present disclosure is not limited thereto.

The "up-down" positional relationship described in the present disclosure is defined by the state where the bottom cover of the buffered drug aerosol delivery device is placed downward on a horizontal surface.

Referring to Figs. 1 and 2, an embodiment of the present disclosure provides a buffered drug aerosol delivery device comprising a chamber 1, a mouthpiece 2, two first pressing buttons 4, a bottom cover 6, two first springs 9, an inhalation controller (such as a flower-shaped washer 10), and an exhalation controller (such as a valve structure 11).

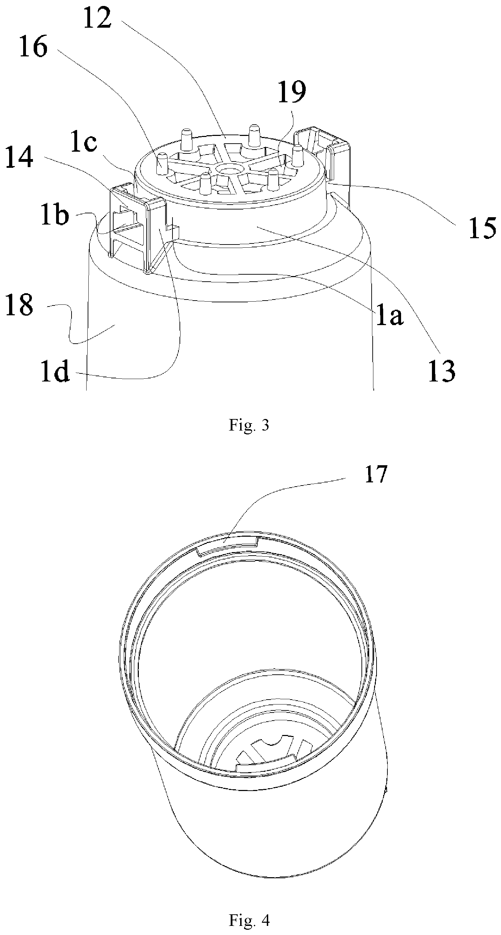

Referring to Figs. 3 and 4, the chamber 1 is an integrally formed structure comprising a tube-shaped portion 18, a stepped portion, two first clip positions 14, and two second clip positions 17.

The tube-shaped portion 18 is tubular with openings at both ends. The first opening of the tube-shaped portion 18 is detachably fixed to the bottom cover 6 configured to receive a metered dose of aerosol from a pressurized metered-dose inhaler. Specifically, the opening of the bottom cover 6 and the outlet of a pressurized metered-dose inhaler match each other, and the outlet of the pressurized metered-dose inhaler is connected to the opening of the bottom cover 6. The tube-shaped portion 18 is not limited to a straight tube, but may be, for example, a curved or stepped tube.

The stepped portion covers the second opening of the tube-shaped portion 18. The central part of the stepped portion protrudes away from the tube-shaped portion 18 to form a boss. The top surface of the boss is a fixing surface 12. The fixing surface 12 is provided with a vent 19 communicating with the hollow region of the tube-shaped portion 18. The outer peripheral surface of the boss serves as a first insertion surface 13.

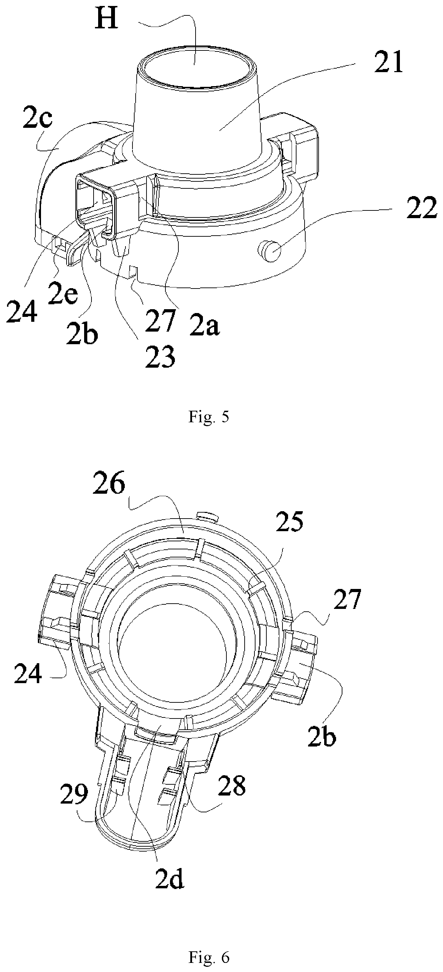

Referring to Figs. 3 and 5, the first clip position 14 is a frame-like structure. The two first clip positions 14 are connected to the first insertion surface 13 (i.e., the outer peripheral surface of the boss which is arranged on the top surface of the stepped portion). The two first clip positions 14 are arranged to be opposed to each other. The top surface of the first clip position 14 comprises a void region (a third notch 1c), and a surface of the first clip position 14 that faces away from the other first clip position 14 comprises a void region (a second notch 1b).

Referring to Figs. 5 and 6, the mouthpiece 2 is an integrally formed structure comprising a main housing and two sleeves 24. The main housing forms a three-stage stepped through hole H. A section of the inner peripheral surface of the main housing that corresponds to the large-diameter stage of the three-stage stepped through hole H constitutes a second insertion surface 26. In the assembled state, the first insertion surface 13 matches the second insertion surface 26.

A section of the main housing that corresponds to the small-diameter stage of the three-stage stepped through hole H constitutes an oral portion 21, to which a user applies his/her mouth. The oral portion 21 may also be connected to a mask (not shown) for a user to breathe with his/her nose.

The two sleeves 24 are tubular with their openings opposed to each other, and a line connecting the centers of the two sleeves 24 goes through the axis of the three-stage stepped through hole H. The first opening of the sleeve 24 is connected to the outer peripheral surface of a section of the main housing that corresponds to the medium-diameter stage of the three-stage stepped through hole H.

Specifically, the sleeve 24 is a rectangular tube. The sleeve 24 is provided, on a section of each of its two side walls opposed to each other in the horizontal direction that is close to the main housing, with a fourth notch 2a.

A fixing rib 15 located below the first notch 1a undetachably links a plate-shaped region 1d of the first clip position 14 to the first insertion surface 13. During assembly, the first positioning rib 23 is inserted between the plate-shaped regions 1d of the first clip position 14 that are opposed to each other in a tangent direction of the first insertion surface 13.

Referring to Figs. 2 and 5, two first springs 9 are in one-to-one correspondence with the two sleeves 24. The first spring 9 is located inside the corresponding sleeve 24, and a first end of the first spring 9 abuts against the outer peripheral surface of a section of the main housing that corresponds to the medium-diameter stage of the three-stage stepped through hole H.

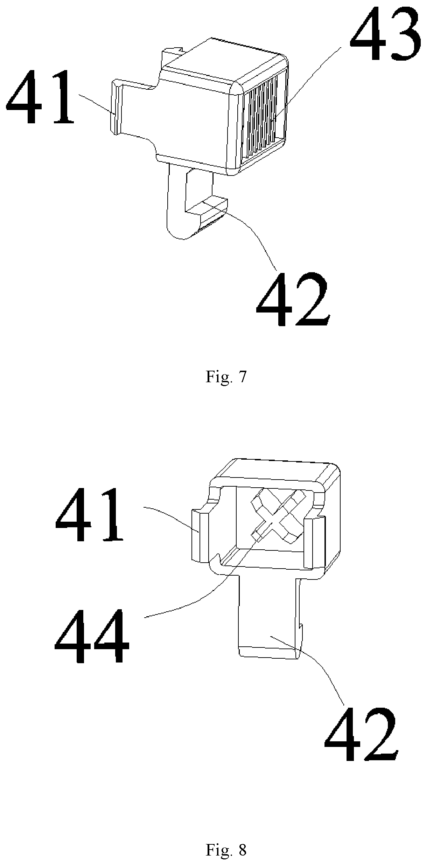

Referring to Figs. 2 and 7, a first pressing button 4 is an integrally molded structure comprising a pressing portion 43, a first clip 42, and two second clips 41. The first pressing buttons 4 are in one-to-one correspondence with the sleeves 24, and the first pressing button 4 is located inside the corresponding sleeve 24 and is movable in the extending direction of the sleeve 24.

A surface of the pressing portion 43 of the first pressing button 4 that faces away from the main housing serves as a pressing surface for a user to press.

A second end of the first spring 9 abuts against a surface of the corresponding first pressing button 4 that faces a section of the main housing that corresponds to the medium-diameter stage of the three-stage stepped through hole H. Referring to Fig. 8, the pressing portion 43 of the first pressing button 4 is provided, on a side surface thereof that faces the main housing, with a positioning rib 44. The second end of the first spring 9 covers the positioning rib 44 to prevent the first spring 9 from swinging. In order for the movement of the first pressing button 4 to be confined to being in the radial direction of the main housing, the main housing is provided, on the outer peripheral surface thereof, with a cross-shaped positioning rib (not shown). The cross-shaped positioning rib on the main housing and the cross-shaped positioning rib 44 of the first pressing button 4 both extend in the radial direction of the main housing, and the extension direction of a line connecting their centers is the radial direction of the main housing.

Shaping the main housing as a 3-stage stepped through hole is for the purpose of reducing the overall size and make the structure compact. If space permits, the main housing may also assume the shape of a 2-stage stepped through hole or the shape of a straight tube.

The bottom wall of the sleeve 24 is provided, at its free end (the end facing away from the main housing), with a fifth notch 2b. The first clip 42 passes through the fifth notch 2b in the bottom section of the sleeve 24 and the third notch 1c on the top surface of the first clip position 14 and enters the internal space of the first clip position 14. The first spring 9 pushes the first pressing button 4 in a direction away from the main housing. The second notch 1b on a surface of the first clip position 14 that faces away from the other first clip position 14 is configured to limit the position of the free end of the first clip 42.

The two second clips 41 extend toward the main housing. When the first spring 9 pushes the first pressing button 4 away from the main housing, the free ends of the two second clips 41 engage with the fourth notches 2a on two side walls of the sleeve 24 that are opposed to each other in a direction parallel to the plane where the main housing is located.

The fixing rib 15 located below the second notch 1b on the first clip position 14 together with a first positioning groove 27 on the end surface of the mouthpiece 2 (the end surface face away from the oral portion 21) prevents a whole consisting of the first pressing button 4 and the mouthpiece 2 from rotating.

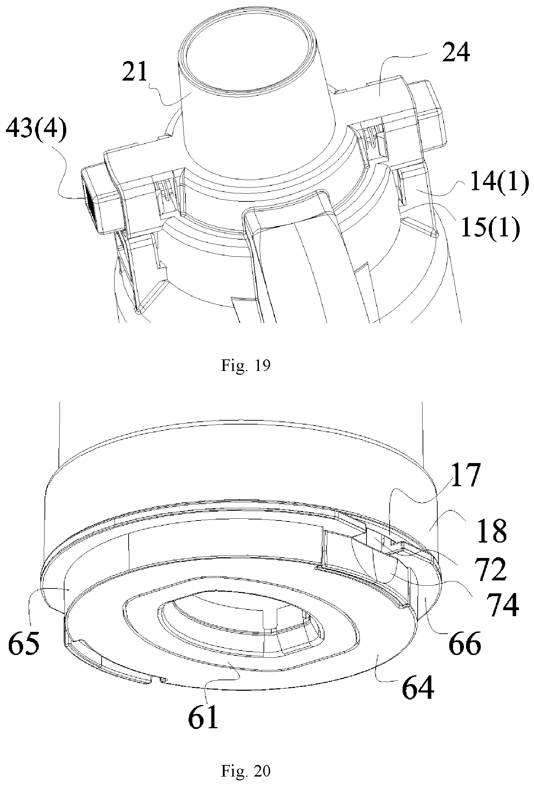

Referring to Fig. 19, during assembly, when the first spring 9 is placed into the sleeve 24, and the two second clips 41 of the first pressing button 4 are inserted into the sleeve 24, the fourth notch 2a on the sleeve 24 engages with the second clip 41, thereby preventing the sleeve 24 from being detached from the first pressing button 4. Then, the chamber 1 is covered with a whole consisting of the mouthpiece 2, the first spring 9, and the first pressing button 4. The first clip 42 of the first pressing button 4 passes through the fifth notch 2b and the third notch 1c and enters the hollow region of the first clip position 14. When the first pressing button 4 is released, the first spring 9 pushes the first pressing button 4 away from the main housing until it arrives at its limit position, and the first clip 42 hooks onto the edge of the second notch 1b, thereby preventing the chamber 1 from being detached from the mouthpiece 2.

Referring to Fig. 19, during disassembly, when the first pressing button 4 is pressed toward the main housing until it reaches its limit position, the end of the first clip 42 disengages from the second notch 1b on the first clip position 14. When the whole consisting of the mouthpiece 2, the first spring 9, and the first pressing button 4 is moved away from the chamber 1 in the axial direction of the tube-shaped portion 18, the first clip 42 of the first pressing button 4 passes through the third notch 1c on the first clip position 14 and disengages from the first clip position 14.

The device features simple assembly and disassembly processes, is structurally steady, and can get its components positioned accurately.

The material of the mouthpiece 2 may be a thermoplastic polymer structural material (such as acrylonitrile-butadiene-styrene copolymer or poly(ethylene glycol-co-1,4-cyclohexanedimethanol terephthalate)).

The material of the chamber 1 may be a thermoplastic polymer structural material (such as acrylonitrile-butadiene-styrene copolymer or poly(ethylene glycol-co-1,4-cyclohexanedimethanol terephthalate)).

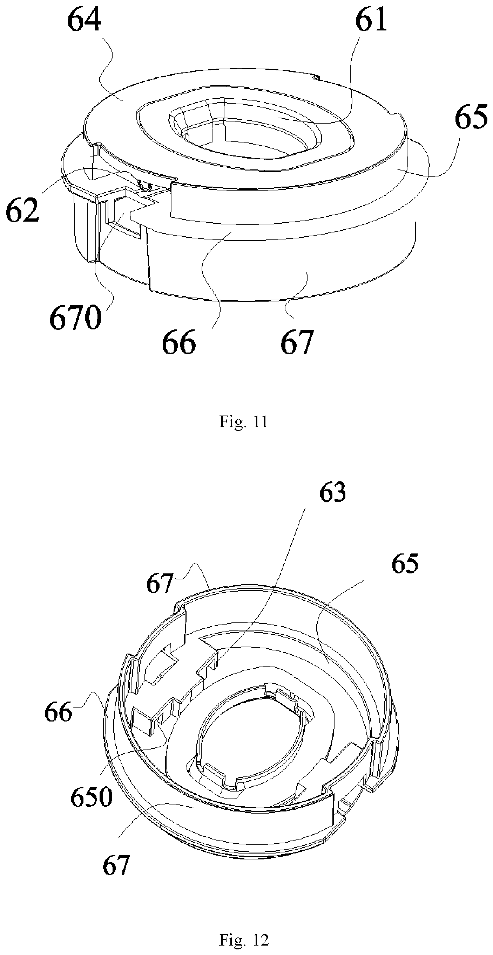

Referring to Figs. 11 and 12, the bottom cover 6 comprises a rigid portion and a soft rubber ring 61. The rigid portion is an integrally formed structure comprising, in this order, a first annular curved plate 67, a first annular flat plate 66, a second annular curved plate 65, and a second annular flat plate 64, which are connected to each other.

The first annular curved plate 67 defines a first cylindrical internal space, and the second annular curved plate 65 defines a second cylindrical internal space. The central opening region of the second annular flat plate 64, the second cylindrical internal space, the central opening region of the first annular flat plate 66, and the first cylindrical internal space communicate, in this order, with each other to form the stepped through hole.

The soft rubber ring 61 is located inside the central opening region of the second annular flat plate 64. The outlet of the pressurized metered-dose inhaler, during its use, is inserted into the soft rubber ring 61.

The second annular curved plate 65 comprises two concave sections. The second annular curved plate 65 is provided, on both sides of the bottom of the concave section thereof, with a first through hole 650. The edge of the first through hole 650 forms a third clip position 63.

The second annular curved plate 65 is provided, at the central section of the concave section thereof, with a second positioning post 62.

The first annular curved plate 67 comprises two concave sections, the first annular flat plate 66 comprises two concave sections, and the second annular flat plate 64 comprises, on its outer periphery, two concave sections. The orthographic projections of the concave sections of the first annular curved plate 67, the concave sections of the first annular flat plate 66, the concave sections of the second annular curved plate 65, and the concave sections of the second annular flat plate 64 onto the plane where the bottom cover 6 is located fall in two centrally symmetric fan-shaped regions. The first annular curved plate 67 is provided, at the boundary of the concave section thereof that faces the first annular flat plate 66, with a sixth notch 670.

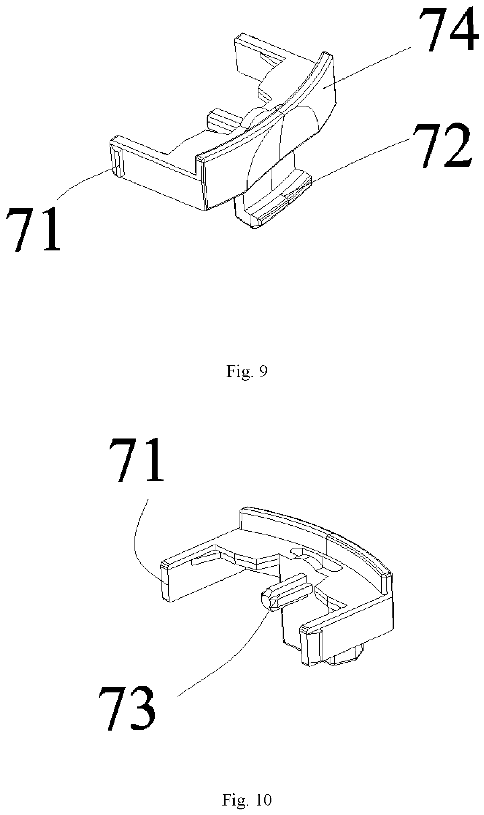

Referring to Figs. 2, 9, and 10, a second pressing button 7 is an integrally formed structure comprising a pressing plate 74, a first positioning post 73, a third clip 72, and two fourth clips 71.

The pressing plate 74 is for a user to press. In this embodiment, the pressing plate 74 is a curved plate, and in other embodiments, the pressing plate 74 may also be a flat plate.

Referring to Figs. 2, 9, and 10, the first positioning post 73 and the two fourth clips 71 are arranged on the inner side surface of the pressing plate 74 (a side surface facing the bottom cover 6). The first positioning post 73 and the two fourth clips 71 extend in the same direction, with the first positioning post 73 located between the two fourth clips 71. The extension direction of the third clip 72 is perpendicular to the plane where the two fourth clips 71 are located.

Referring to Fig. 4, the tube-shaped portion 18 is provided, at the first opening thereof, with the two second clip positions 17 opposed to each other. The two second clip positions 17 are arc-shaped flat plates, with their normal directions substantially being the extension direction of the tube-shaped portion 18.

Referring to Fig. 2, the buffered drug aerosol delivery device further comprises two second springs 8 that are in one-to-one correspondence with the two second annular curved plates 65.

The first end of the second spring 8 covers the second positioning post 62 on the corresponding second annular curved plate 65 and abuts against the concave section of the second annular curved plate 65. The second end of the second spring 8 covers the first positioning post 73 of the corresponding second pressing button 7 and abuts against the inner side surface of the pressing plate 74 of the corresponding second pressing button 7.

The two fourth clips 71 pass through the first through holes 650 in the concave sections of the second annular curved plates 65, respectively, and engage with the edges (i.e., the third clip positions 63) of the first through holes 650.

The second clip position 17 is opposed to the concave section of the first annular flat plate 66 and abuts against a side surface of the first annular flat plate 66 that faces the first annular curved plate 67. The end of the third clip 72 engages with a side surface of the second clip position 17 that faces away from the first annular flat plate 66.

The concave section of the first annular curved plate 67 serves to limit the position of the second clip position 17, preventing the second clip position 17 from rotating with respect to the bottom cover 6.

Referring to Fig. 20, during assembly, when the second positioning post 62 on the second annular curved plate 65 is covered with the first end of the second spring 8, and the fourth clip 71 is inserted into the first through hole 650 of the second annular curved plate 65, the fourth clip 71 gets its position limited by the third clip position 63, which prevents the second pressing button 7 from be detached radially from the bottom cover 6. At this time, the pressing plate 74 is located on a side surface of the first annular flat plate 66 that faces the second annular flat plate 64, keeping the second spring 8 in a compressed state. The first annular curved plate 67 is inserted into the first opening of the tube-shaped portion 18, and the second clip position 17 is at the same time caused to abut against a side surface of the first annular flat plate 66 that faces the first annular curved plate 67. The second spring 8 is then released, which pushes the third clip 72 outward and causes the end of the third clip 72 to engage with a side surface of the second clip position 17 that faces away from the first annular flat plate 66.

During disassembly, pressing the pressing plate 74 of the second pressing button 7 drives the third clip 72 to move radially inward. As a result, the end of the third clip 72 releases the second clip position 17, allowing the whole consisting of the second pressing button 7, the second spring 8, and the bottom cover 6 to disengage from the tube-shaped portion 18.

The inhalation controller is detachably connected to the chamber and is configured to control the gas such that it flows unidirectionally into the mouthpiece from the interior of the chamber through the first vent 19.

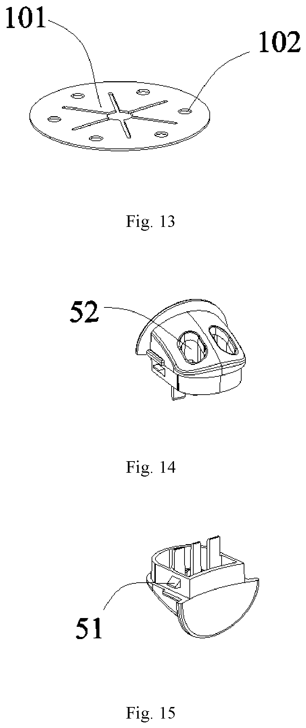

Specifically, referring to Figs. 3 and 13, the inhalation controller is a flower-shaped washer 10 comprising a plurality of petals 101 and a plurality of positioning holes 102 distributed over the petals 101. The fixing surface 12 is provided with a plurality of first vents 19 and a plurality of first fixing posts 16. The plurality of first fixing posts 16 are inserted into the plurality of positioning holes 102 in a one-to-one correspondence way, and the plurality of petals 101 cover the plurality of first vents 19 in a one-to-one correspondence way.

During inhalation, a side of the flower-shaped washer 10 that faces away from the fixing surface 12 is under negative pressure, causing the flower-shaped washer 10 to separate from the fixing surface 12 and thereby allowing the aerosol to enter the mouthpiece from the chamber 1.

The exhalation controller is undetachably connected to the mouthpiece 2 and is configured to control the gas such that it unidirectionally flows out of the buffered drug aerosol delivery device from the mouthpiece 2 through the exhalation controller.

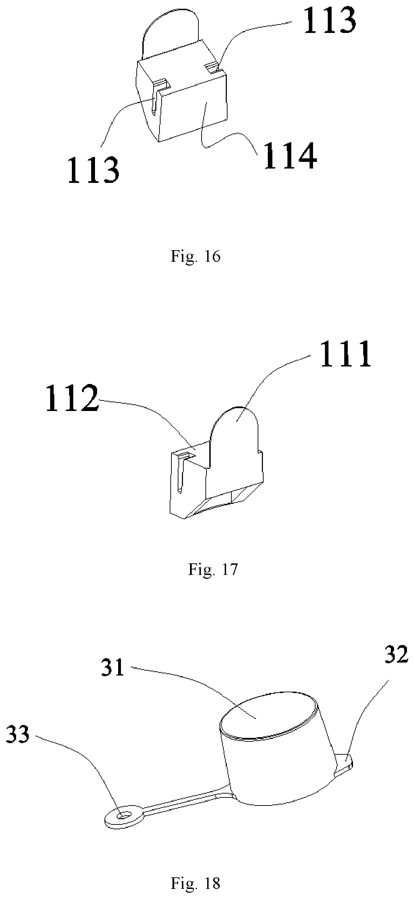

Specifically, referring to Figs. 2, 5, 6, 16, and 17, the exhalation controller is a valve structure 11 made of an integrally formed elastic material and comprising a valve flap 111 and a second positioning groove 113. The second positioning groove 113 is arranged on a stepped surface 112, and the valve flap 111 is located at the edge of the stepped surface 112.

Referring to Fig. 20, during assembly, a second positioning rib 29 is inserted into the second positioning groove 113. Fig. 6 shows four second positioning ribs 29, two of which are spare.

Specifically, referring to Fig. 5, the mouthpiece 2 further comprises an extension portion 2c extending radially outward from the main housing. The main housing is provided, on a section of the surface thereof that corresponds to the large-diameter stage of the three-stage stepped through hole H, with a second vent 2d, and the valve flap 111 covers the second vent 2d.

During exhalation, the increased air pressure inside the mouthpiece 2 pushes the valve flap 111 away from the second vent 2d, thereby allowing air to exit the mouthpiece 2.

During exhalation, the flower-shaped washer 10 presses the fixing surface 12, thereby blocking the first vents 19 on the fixing surface 12. During inhalation, the valve flap 111 blocks the second vent 2d.

A positioning boss 28 abuts against the stepped surface 112 of the valve structure 11 to limit the position of the valve structure 11. A mounting surface 114 of the valve structure 11 abuts against the outer peripheral surface of a section of the main housing that corresponds to the medium-diameter stage of the three-stage stepped through hole H.

Referring to Fig. 6, the mouthpiece 2 is provided with a vertical rib 25. When the device is in the assembled state, the vertical rib 25 presses the flower-shaped washer 10 to prevent it from being detached from the fixing surface 12.

The main housing is provided, on an end surface thereof that faces the chamber 1, with a first positioning groove 27. When the device is in the assembled state, the first positioning groove 27 is inserted into the first notch 1a.

Optionally, the device further comprises a mouthpiece clip 5 undetachably connected to the extension portion 2c and providing a gas passage from the second vent 2d to the exterior of the buffered drug aerosol delivery device.

Referring to Figs. 14 and 15, the mouthpiece clip 5 comprises a fifth clip 51 and an exhaust hole 52. During exhalation, the second vent 2d communicates with the exhaust hole 52 via the valve structure 11. The fifth clip 51 snaps into a second through hole 2e formed on the extension portion 2c.

Based on the same inventive concept, the present disclosure further provides a buffered drug aerosol delivery system comprising the buffered drug aerosol delivery device described above and a pressurized metered-dose inhaler. The outlet of the pressurized metered-dose inhaler is inserted into an opening at the center of the bottom cover 6 of the buffered drug aerosol delivery device and thereby delivers a metered dose of aerosol into the chamber 1.

The first clip 42, second clip 41, third clip 73, and fourth clip 71 each comprise a strip-shaped flat plate region and a corner region at the free end of the flat plate region. The two second clips 41 as well as the two fourth clips 71 form a split pin structure.

Referring to Figs. 1, 2, 5, and 18, the buffered drug aerosol delivery device further comprises a dust cap 3. The dust cap 3 comprises a cap body 31, a cap visor 32, and a fixing female snap 33. When the device is in use, the fixing female snap 33 engages with a fixing male snap 22 on the mouthpiece 2 to prevent the dust cap 3 from being lost. The cap visor 32 allows the user to conveniently remove the dust cap 3. The cap body 31 covers the oral portion 21.

In some specific embodiments, the material of the dust cap 3 is, for instance, soft thermoplastic rubber (TPR). The material of the mouthpiece 2 is, for example, acrylonitrile-butadiene-styrene (ABS) or poly(ethylene glycol-co-1,4-cyclohexanedimethanol terephthalate) (PETG). The material of the valve structure 11 is, for example, silicone. The material of the mouthpiece clip 5 is, for example, ABS or PETG. The material of the flower-shaped washer 10 is, for example, silicone. The material of the first pressing button 4 is, for example, ABS or PETG. The material of the chamber 1 is, for example, ABS or PETG. The material of the second pressing button 7 is ABS. The rigid portion of the bottom cover 6 is made of rigid ABS, while the soft rubber ring 61 of the bottom cover 6 is made of soft TPR.

Each embodiment in the present disclosure is described in a progressive manner. Identical or similar parts between embodiments can refer to each other. Each embodiment is focused on its differences from other embodiments.

The scope of protection of the present disclosure is not limited to the above embodiments. It is evident that one skilled in the art would be able to modify and transform the present disclosure in different ways without departing from its scope and spirit. If such modification and transformation fall within the scope of the claims of the present disclosure or the scope of their equivalents, then the present disclosure is also intended to encompass the modification and transformation.

## Claims

1. A buffered drug aerosol delivery device, comprising: a chamber, a mouthpiece, two first pressing buttons, two first springs, a bottom cover, an inhalation controller, and an exhalation controller;
wherein the first pressing button is an integrally formed structure comprising a pressing portion, a first clip, two second clips, and a positioning structure, wherein the first clip is connected to the pressing portion and extends downward, and the two second clips and the positioning structure are all connected to the pressing portion and extend horizontally toward the interior of the buffered drug aerosol delivery device;
wherein the chamber is an integrally formed structure providing a gas passage extending in an up-down direction and comprising two first clip positions arranged on an outer side of the gas passage, wherein the two first clip positions are opposed to each other in a diameter direction of the chamber and are hollow, and a hollow region of the first clip position extends upward and away from the opposed first clip position;
wherein the mouthpiece is an integrally formed structure comprising a main housing and two sleeves, wherein the main housing provides a gas passage extending in an up-down direction, and the two sleeves are arranged on an outer side of the main housing and extend in a diameter direction of the main housing with their respective openings opposed to each other;
wherein the first pressing button, the first clip position, the sleeve, and the first spring are in one-to-one correspondence with each other;
wherein the first spring is located inside the corresponding sleeve, and two ends of the first spring are connected to the main housing of the mouthpiece and the positioning structure of the first pressing button, respectively;
wherein the pressing portion of the first pressing button is located inside the corresponding sleeve, wherein the sleeve is provided, at a bottom thereof, with a void region extending to an end surface thereof that faces away from the other sleeve to allow and restrict the movement of the first pressing button in an extension direction of the corresponding sleeve, and the sleeve is provided, at an end thereof that faces the other sleeve, with two void regions opposed to each other horizontally to secure free ends of the two second clips of the corresponding first pressing button;
wherein when the first pressing button is at a farthest position from the other first pressing button, a free end of the first clip of the first pressing button engages with an upper boundary of the first clip position that faces away from the void region on the end surface of the other first clip position, and when the first pressing button is at a closest position to the other first pressing button, the first clip of the first pressing button is movable to be detached from the first clip position; and
wherein the bottom cover is detachably fixed to a lower end of the chamber, the inhalation controller is configured to allow gas to flow unidirectionally out of the mouthpiece from the chamber through the main housing of the mouthpiece, and the exhalation controller is configured to allow gas to flow unidirectionally out of the mouthpiece from an upper opening of the main housing of the mouthpiece through an internal space of the main housing of the mouthpiece.

2. The buffered drug aerosol delivery device according to claim 1, wherein the chamber comprises a tube-shaped portion and a stepped portion, wherein the tube-shaped portion is tubular and comprises a first opening detachably fixed to the bottom cover, the stepped portion covers a second opening of the tube-shaped portion and comprises a central region protruding upward to form a boss provided, on a top surface thereof, with a first vent communicating with an internal space of the tube-shaped portion, and the first clip position is connected to the stepped portion and configured to be a frame-like structure, is provided, at an upper part thereof, with a third notch, and is provided, on a side thereof that faces away from the boss, with a second notch, wherein the third notch and the second notch communicate with an internal space of the first clip position; and
the main housing of the mouthpiece defines a three-stage stepped through hole, wherein the sleeve of the mouthpiece is opposed to a medium-diameter stage of the three-stage stepped through hole, and the boss of the stepped portion of the chamber is fitted into a section of the main housing that corresponds to the large-diameter stage of the three-stage stepped through hole.

3. The buffered drug aerosol delivery device according to claim 1, wherein the positioning structure of the first pressing button is a cross-shaped positioning rib.

4. The buffered drug aerosol delivery device according to claim 1, wherein the mouthpiece further comprises a first positioning rib connected to the bottom of the sleeve, wherein the first positioning rib extends downward and is inserted into the hollow region of the first clip position.

5. The buffered drug aerosol delivery device according to claim 2, wherein the mouthpiece is provided, at a lower end surface thereof, with a first positioning groove, and the first clip position is connected to a bottom peripheral region of the boss via a fixing rib, wherein a first notch is formed above the fixing rib, and the first positioning groove is mounted onto the fixing rib by passing through the first notch.

6. The buffered drug aerosol delivery device according to claim 2, further comprising two second pressing buttons and two second springs;
wherein the second pressing button is an integrally formed structure comprising a pressing plate, a third clip, two fourth clips, and a first positioning post, wherein the first positioning post and the fourth clips are located on the same side of the pressing plate and extend horizontally toward the interior of the buffered drug aerosol delivery device, and the third clip extends upward;
the chamber further comprises two second clip positions located at the first opening of the tube-shaped portion thereof, wherein the second clip position is an arc-shaped flat plate;
the bottom cover comprises an integrally formed rigid portion comprising four first through holes for insertion of the fourth clip of the second pressing button, wherein an edge of the first through hole forms a third clip position configured to prevent the fourth clip from being detached from the bottom cover, and the rigid portion of the bottom cover further comprises two second positioning posts arranged between adjacent ones of the first through holes and extending radially outward;
the second pressing button, the second spring, and the second positioning post are in one-to-one correspondence with each other;
two ends of the second spring cover the corresponding second positioning post and the first positioning post of the corresponding second pressing button respectively and abut against the bottom cover and the pressing plate of the second pressing button;
a free end of the third clip of the second pressing button engages with the second clip position to prevent the bottom cover from being detached axially from the tube-shaped portion; and
the bottom cover comprises, on the outer peripheral surface of a section thereof that faces the tube-shaped portion, two concave sections, wherein the second clip position is located between the two concave sections to prevent the bottom cover from rotating with respect to the tube-shaped portion.

7. The buffered drug aerosol delivery device according to claim 6, wherein the bottom cover comprises a first annular curved plate, a first annular flat plate, a second annular curved plate, and a second annular flat plate, connected in this order from top to bottom, wherein the first annular curved plate defines a first cylindrical internal space, the second annular curved plate defines a second cylindrical internal space, and a central opening region of the second annular flat plate, the second cylindrical internal space, a central opening region of the first annular flat plate, and the first cylindrical internal space communicate, in this order, with each other to form a through hole;
the second annular curved plate comprises two opposed concave sections in one-to-one correspondence with the second pressing buttons, wherein the pressing plate of the second pressing button is opposed to the corresponding concave section of the second annular curved plate, and the first through hole into which the fourth clip of the second pressing button is inserted is arranged at the concave section of the second annular curved plate;
the first annular flat plate comprises two opposed concave sections in one-to-one correspondence with the second pressing buttons, wherein the concave section of the first annular flat plate is configured to avoid the third clip of the second pressing button; and
the first annular curved plate is inserted into the first opening of the tube-shaped portion and comprises two opposed concave sections in one-to-one correspondence with the second pressing buttons, wherein the second clip position is located inside the corresponding concave section of the first annular curved plate to prevent the bottom cover from rotating with respect to the tube-shaped portion.

8. The buffered drug aerosol delivery device according to claim 2, wherein the inhalation controller is a flower-shaped washer arranged on a top surface of the boss of the stepped portion.

9. The buffered drug aerosol delivery device according to claim 1, wherein the exhalation controller is a valve structure, and the main housing of the mouthpiece is provided with a second vent, wherein the valve structure comprises an elastic valve flap configured to cover the second vent.

10. A buffered drug aerosol delivery system, comprising a pressurized metered-dose inhaler and the buffered drug aerosol delivery device according to any one of claims 1 to 9.
